# EUROPÄISCHE PATENTANMELDUNG

(11) **EP 0 860 701 A1**
(43) Veröffentlichungstag der Anmeldung: **26.08.1998**
(21) Anmeldenummer: 98102919.2
(22) Anmeldetag: 19.02.1998
(51) Int. Cl.: G01N 33/94, G01N 33/543

(54) **Teststrip combination**

(30) Priorität: 19.02.1997 DE 29702825 U; 28.01.1998 DE 29801335 U
(71) Anmelder: "The Ultimate" Pharma et Health Products GmbH, 22926 Ahrensburg (DE)
(72) Erfinder: Die Erfindernennung liegt noch nicht vor
(74) Vertreter: Siewers, Gescha, Dr.

(57) **Zusammenfassung**

Die Erfindung betrifft eine Teststreifenkombination zum Nachweis von Fremdsubstanzen in Körperflüssigkeiten, insbesondere Urin, wobei vorzugsweise die Teststreifen in einem Teststreifenhalter eingesetzt sind und wobei mehrere Teststreifen in passende Aussparungen des Halters so befestigt sind, daß die Teststreifen mit dem in die Probe zu tauchenden Ende aus dem Halter hervorstehen, und wobei der Halter mit einer einseitigen Klebefolie zur Fixierung der Teststreifen im Halter abgedeckt ist und die Klebefolie wengistens im Bereich der Nachweisfelder der Teststreifen transparent ist, und ist dadurch gekennzeichnet, daß zusätzlich mindestens ein Teststreifen zum Nachweis des Normalgehaltes an bestimmten Bestandteilen der Körperflussigkeit vorgesehen ist.

## Beschreibung

Die Neuerung betrifft Teststreifenkombinationen zum Nachweis von Fremdsubstanzen in Körperflüssigkeiten.

Teststreifen zum Nachweis von Fremdsubstanzen oder deren Metaboliten in Körperflüssigkeiten wie Speichel, Plasma oder meist Urin sind an sich bekannt und werden in großem Umfang zum schnellen Screening oder zur Kontrolle im klinischen und auch kriminaltechnischen Bereich eingesetzt. Da die meisten suchterzeugenden und/oder das Verhalten, insbesondere die Fahrtüchtigkeit des Menschen beeinflussenden Substanzgruppen harnpflichtig sind, wie beispielsweise Morphinabkömmlinge, synthetische stark wirkende Analgetika, wie Pethidin, Barbiturate und Benzodiazepine, aber auch Lysergsäure- oder Phenylpropanderivate, wird für derartige Schnelltests meist der Urin des Probanden eingesetzt. Kontrollen dieser Art werden daher durchgeführt bei Schnellkontrollen unter ärztlicher Aufsicht im Anschluß an Verkehrskontrollen und auffälligem Verkehrsverhalten von Fahrern, aber auch zur Überprüfung von Beteiligten in Drogenentziehungs- oder Drogensubstitutionsprogrammen und in einigen Ländern auch zur Kontrolle einer möglichen Drogenabhängigkeit bei Personen in Berufen mit einem hohen potentiellen Gefährdungspotential für die Allgemeinheit, wie beispielsweise Bedienungsmannschaften bei Kernkraftwerken, bei Luftlinien usw.

Verständlicherweise versuchen Abhängige ihren Drogenkonsum bei derartigen Kontrollen soweit wie möglich zu kaschieren und Manipulationsmöglichkeiten sind insbesondere im Gegensatz zu Speichel- oder Plasmaanalysen bei Urinanalysen gegeben. Die Empfindlichkeit der Nachweisreaktionen auf Fremdsubstanzen oder deren Metaboliten ist bei Schnelltests in der Regel so ausgelegt, daß die Farb- oder Fluoressenzreaktionen erst bei einem normalerweise zu erwartenden Gehalt an zu analysierender Substanz ansprechen, so daß versucht wird, durch Verdünnen von Urinproben mit Wasser in einen Bereich zu gelangen, in dem die Reaktion nicht mehr anspricht oder, wenn quantitativ ausgeführt, ein deutlich zu niedriges Ergebnis anzeigt. In den einschlägigen Kreisen ist auch bekannt, daß solche Nachweisreaktionen durch Verschieben des pH-Wertes; wie beispielsweise durch Zusatz von Seifenlösungen; oder durch Zugabe von stark reduzierenden Substanzen, wie beispielsweise Glukose, entweder unterbunden oder in ihrem Aussagewert verändert bis unbrauchbar gemacht werden können.

Es besteht daher noch ein Bedarf an Teststreifenkombinationen zum Nachweis von Fremdsubstanzen in Körperflüssigkeiten und insbesondere im Urin, die gleichzeitig eine Aussage darüber erlauben, ob Manipulationen an der zu untersuchenden Probe vorgenommen wurden.

Zur Lösung wird eine Teststreifenkombination vorgeschlagen, die dadurch gekennzeichnet ist, daß mindestens ein Teststreifen zum Nachweis des Normalgehaltes an bestimmten Bestandteilen der Körperflüssigkeit und insbesondere des Urins geeignet ist.

Der Gehalt des menschlichen Urins an organischen und anorganischen Stoffen unterliegt zwar physiologischen Schwankungen, aber beim gesunden Erwachsenen wird eine gewisse Bandbreite an den Inhaltsstoffen regelmäßig nicht über- oder unterschritten. Der pH-Wert des frischen Urins beträgt etwa 5,0 bis 6,4. Während der Gehalt beispielsweise an Harnstoff und reduzierenden Verbindungen in Abhängigkeit von der Ernährung etwas stärker schwanken können, gibt es bei anderen Substanzen nur eine relativ konstante Bandbreite. Ein gesunder Erwachsener scheidet, bezogen auf den 24-Stunden-Harn, etwa 0,5 g Harnsäure und etwa 1,2 g Kreatinin aus, die sich als Indikatorsubstanz verwenden lassen. Auch der Gehalt des Harns an Urobilinogenen, also Abbauprodukte des Bilinogens, ist relativ konstant und kann zur halbquantitativen Bestimmung eingesetzt werden.

Farbreaktionen zum Nachweis dieser oder anderer obligatorisch im Harn vorhandener Verbindungen, auch in halb- oder ganzquantitativer Form, sind dem Fachmann bekannt; wir verweisen beispielsweise auf Sonntag, Trockenchemie, Analytik mit trägergebundenen Reagenzien, Thieme-Verlag, Stuttgart, 1988.

Die verwendeten Teststreifen sind entweder imprägnierte Papierstreifen, häufig mit einer Kunststoffunterlage zur Verbesserung der mechanischen Eigenschaften oder Kunststoffolien, auf denen die Reagenzien, Indikatoren, Puffer und Maskierungssubstanzen gebunden sind. Bei Teststreifen dieser Art dient das in der Körperflüssigkeit enthaltenene Wasser als Lösungsmittel und Schlepper, das die nachzuweisende Verbindung zu dem Reagenz transportiert, das in der Regel in der Verbindung eine Reaktion im sichtbaren Bereich eingeht oder durch Fluoressenz nachweisbare Reaktionsprodukte bildet. Teststreifen können so ausgelegt sein, daß mehrere Reaktionszonen vorhanden sind, so daß also durch einen Versuch gleich mehrere Substanzen nachgewiesen werden können. Vorzugsweise werden Teststreifen zu Nachweisreaktionen der hier geforderten Art als Kombination eingesetzt, in dem mehrere Streifen entweder durch einen Papier- oder Kunststoffolienstreifen miteinander verbunden oder vorzugsweise in einem Teststreifenhalter entsprechend dem deutschen Gebrauchsmuster 297 02 825 eingesetzt sind. Derartige Teststreifenhalter sind so aufgebaut, daß einer oder mehrere Testreifen in passende Aussparungen des Halters so eingelegt oder eingeklebt sind, daß die Teststreifen mit dem in die Probe zu tauchenden Ende aus dem Halter hervorstehen, wobei der Halter selbst in der Regel eine Kunststoffplatte mit streifenförmigen Vertiefungen in der Breite der Teststreifen ist. Diese Halter sind vorzugsweise auch im Bereich der Aussparungen mit einer einseitigen Klebefolie abgedeckt, die einerseits zur Fixierung der Teststreifen am Halter dient, andererseits aber die Teststreifen vor Verunreinigungen oder Beschädigungen schützt. Diese Klebefolie ist wenigstens im Bereich der Nachweisfelder der Streifen transparent ausgeführt, so daR Farbreaktionen ohne Schwierigkeiten erkennbar sind. Außerdem sind die Klebefolien beschriftet oder jedenfalls beschriftbar.

Im vorliegenden Falle ist eine solche Teststreifenkombination so aufgebaut, daR mindestens ein Teststreifen anzeigt, daß die zu untersuchende Körperflüssigkeit, insbesondere also Urin, unverändert und nicht manipuliert ist. Beispielsweise zeigt ein deutlich über pH 7 liegender Wert an, daß dem Original-Urin Seifenlösung oder andere alkalisch reagierende Substanzen zugesetzt wurden, um durch eine Verschiebung des pH-Wertes die in der Regel im schwachsauren Bereich ablaufenden Farbreaktionen zu stören. Ein zu geringer Gehalt an Kreatinin, Harnsäure oder Urobilinogen beweist eindeutig, daß die Probe mit Wasser oder anderen neutral reagierenden Lösungsmitteln verdünnt wurde, um die Nachweisgrenzen zu unterschreiten.

Die Erfindung wird im folgenden anhand eines Beispiels näher dargelegt:

In einem Teststreifenhalter mit Vorrichtungen zur Aufnahme von drei Teststreifen gemäß deutschem Gebrauchsmuster 297 02 825 sind drei Teststreifen befestigt, von denen der erste dazu bestimmt ist, den Nachweis des Normalgehaltes eines Urins eines Probanden an Kreatinin und den pH-Wert der Lösung anzugeben. Die beiden weiteren Teststreifen sind in bekannter Weise mit Nachweisreagenzien für Heroin bzw. dessen Metaboliten Morphin bzw. für Cocain als den zur Zeit gängigsten Rauschdrogen versehen.

## Patentansprüche

1. Teststreifenkombination zum Nachweis von Fremdsubstanzen in Körperflüssigkeiten, dadurch gekennzeichnet, daß zusätzlich mindestens ein Teststreifen zum Nachweis des Normalgehaltes an bestimmten Bestandteilen der Körperflüssigkeit vorgesehen ist.

2. Teststreifenkombination nach Anspruch 1, dadurch gekennzeichnet, daß sie zur Untersuchung von Urin bestimmt ist.

3. Teststreifenkombination nach Anspruch 1 oder 2, dadurch gekennzeichnet, daß der oder die Teststreifen zur Feststellung des Normalgehaltes an bestimmten Bestandteilen der Körperflüssigkeit mehrere Parameter gleichzeitig anzeigen.

4. Teststreifenkombination nach Anspruch 1 bis 3, dadurch gekennzeichnet, daß der oder die Teststreifen pH-Wert, Kreatinin, Harnsäure und/oder Urobilinogen anzeigen.

5. Teststreifenkombination nach Anspruch 1 bis 4, dadurch gekennzeichnet, daß die Teststreifen in einem Teststreifenhalter eingesetzt sind, wobei mehrere Teststreifen in passende Aussparungen des Halters so befestigt sind, daß die Teststreifen mit dem in die Probe zu tauchenden Ende aus dem Halter hervorstehen, daß der Halter mit einer einseitigen Klebefolie zur Fixierung der Teststreifen im Halter abgedeckt ist und daß die Klebefolie wenigstens im Bereich der Nachweisfelder der Teststreifen transparent ist.
